# EUROPEAN PATENT SPECIFICATION

(11) **EP 1 221 865 B1**
(45) Date of publication and mention of the grant of the patent: **03.01.2007**
(21) Application number: 00969121.3
(22) Date of filing: 18.10.2000
(51) Int. Cl.: A23L 1/0528, A23L 1/30, A23C 9/15, A61K 47/10, A61K 31/195

(54) **FOOD SUPPLEMENT FOR INCREASING LEAN MASS AND STRENGTH**
NAHRUNGSMITTELZUSATZ ZUR ZUNAHME DER FETTARMEN KÖRPERMASSE UND KRAFT
COMPLEMENT ALIMENTAIRE DESTINE A AUGMENTER LA MASSE MAIGRE ET LA RESISTANCE DE CELLE-CI

(30) Priority: 18.10.1999 US 420439; 13.01.2000 US 482688
(43) Date of publication of application: 17.07.2002
(73) Proprietor: Muscletech Research and Development Inc., Mississauga, Ontario L5S 1V7 (CA)
(72) Inventor: GARDINER, Paul T., Brampton, Ontario L6W 4S6 (CA); WOODGATE, Derek, E., Brampton, Ontario, L6W 4K4 (CA); GILBERT, Mark, S., Oakville, Ontario L6M 2P4 (CA); THOBURN, Robert, W., Toronto, Ontario M5A 4P1 (CA)
(74) Representative: Gulde, Klaus W.
(86) International application number: PCT/CA2000/001207
(87) International publication number: WO 2001/028356

(56) References cited:
- WO-A2-01/56402
- US-A- 5 891 441
- DATABASE FSTA [Online] INTERNATIONAL FOOD INFORMATION SERVICE (IFIS), FRANFURT/MAIN, DE; HAGLIND C ET AL: "Effects of caffeine containing energy drinks." Database accession no. 2000-00-h0904 XP002164660 & SCANDINAVIAN JOURNAL OF NUTRITION 43 (4) 169-175 1999 SPORTS CLUB, STAVGRAND 16, 129 48 HAGERSTEN, SWEDEN, XP000997463
- DATABASE WPI Section Ch, Week 199918 Derwent Publications Ltd., London, GB; Class D13, AN 1999-205425 XP002164989 & CN 1 201 599 A (LI P)
- PATENT ABSTRACTS OF JAPAN vol. 009, no. 184 (C-294), 30 July 1985 (1985-07-30) & JP 60 054321 A (CHIYODA YAKUHIN KK), 28 March 1985 (1985-03-28)
- PATENT ABSTRACTS OF JAPAN vol. 012, no. 353 (C-530), 21 September 1988 (1988-09-21) & JP 63 109736 A (KYODO NYUGYO KK;OTHERS: 01), 14 May 1988 (1988-05-14)
- PATENT ABSTRACTS OF JAPAN vol. 013, no. 156 (C-585), 14 April 1989 (1989-04-14) & JP 63 310827 A (SANWA KAGAKU KENKYUSHO CO LTD), 19 December 1988 (1988-12-19)
- DATABASE BIOSIS [Online] BIOSCIENCES INFORMATION SERVICE, PHILADELPHIA, PA, US; 1993 BELTZ SUSAN DURDEN ET AL: "Efficacy of nutritional supplements used by athletes." Database accession no. PREV199497073408 XP002164661 & CLINICAL PHARMACY, vol. 12, no. 12, 1993, pages 900-908, ISSN: 0278-2677
- DATABASE BIOSIS [Online] BIOSCIENCES INFORMATION SERVICE, PHILADELPHIA, PA, US; 1995 WILLIAMS MELVIN H: "Nutritional ergogenics in athletics." Database accession no. PREV199598427981 XP002164662 & JOURNAL OF SPORTS SCIENCES, vol. 13, no. SPEC. ISSUE, 1995, pages S63-S74, ISSN: 0264-0414

## Description

### FIELD OF INVENTION

The present invention is directed to food supplements which comprise a substance which increases nitric oxid production in the body selected from the group consisting of ginseng, 1-arginine, N-acetyl cysteine, glucomannan and folic acid and a source of amino acids or derivatives thereof which is a protein; and further a compound which enhances and mimics insulin activity which is selected from the group consisting of myo-inositol, d-myo-inositol, cis-inositol, epi-inositol, allo-inositol, muco-inositol, neo-inositol, scyllo-inositol, d-chiro-inositol, 1-chiro-inositol and d-pinitol and their use for supplementing the diet of an athlete and for enhancing an athlete's muscle size and/or strength.

### BACKGROUND OF INVENTION

Food supplements for enhancing an athlete's muscle size and strength have become popular substitutes for steroids and other drugs in various sports and body building regimes. However, as athletes continually strive for improved performance, there is a continuing need for non-steroid containing aids for increasing lean mass, muscle size and strength.

In DATABASE WPI Section Ch, Week 199918 Derwent Publications Ltd., London, GB; Class D13, AN 1999-205425 XP002164989 & CN 1 201 599 A a food is disclosed which comprises pumkin powder and konjak powder, preferred with corn flour and bean powder and further preferred is an addition of American ginseng powder and others. Furthermore in Patent Abstracts of Japan vol. 012, no. 353 (C-530), 21 September 1988 (1988-09-21) & JP 63 109736 A a solid food is described which contains whey protein and for example glucomannan as an additive.

### SUMMARY OF THE INVENTION

Nitric oxide plays an essential role in tonic and exercise-associated (e.g., recovery from exercise) regulation of vasodilation and blood flow. The present inventors have found that increased NO through supplementing the diet of an athlete with substances which increase the concentration of nitric oxide (NO) in the body selected from the group consisting of ginseng, l-arginine, N-acetyl cysteine, glucomannan and folic acid, in combination with a protein as a source of amino acids in combination with a substance which may increase insulin sensitivity, and which is selected from the group consisting of myo-inositol, d-myo-inositol, cis-inositol, epi-inositol, allo-inositol, muco-inositol, neo-inositol, scyllo-inositol, d-chiro-inositol, 1-chiro-inositol and d-pinitol, may provide surprising enhancement of an athlete's muscle size and/or strength when administered to an athlete's diet. Accordingly, in one broad aspect the present invention provides new food supplements to increase the delivery and duration of nitric oxide in the body. These food supplements do not themselves contain nitric oxide; rather, they act to promote the production of nitric oxide in the body. Preferably the invention provides the use of the named food supplements particularly adapted for supplementing the diet of an athlete, preferably the use for enhance an athlete's muscle size and/or strength.

According to yet another aspect of the present invention, there is provided the said supplement which may increase nitric oxide and thereby increase nitrogen retention in the body.

The supplement of the invention comprises as a preferred substance which increases nitric oxide production ginseng, 1-arginine, N-acetyl cysteine, or folic acid, preferably ginseng.

Furthermore the said food supplement of the invention comprises a substance which provides at least one function selected from the group consisting of enhancing insulin activity and mimicking insulin activity selected from myo-inositol, d-myo-inositol, cis-inositol, epi-inositol, allo-inositol, muco-inositol, neo-inositol, scyllo-inositol, d-chiro-inositol, 1-chiro-inositol, or d-pinitol, in this respect, preferably the substance is myo-inositol.

A preferred substance which increases nitrogen retention in the body is glucomannan or 1-arginine, most preferably glucomannan.

According the invention, the preferred source of amino acids in the supplement comprises whey protein or a whey protein derivative, preferably the whey protein derivative, more preferably two or more whey protein derivatives.

As already mentioned, the substance which increases nitric oxide production is ginseng, 1-arginine, N-acetyl cysteine or folic acid, preferably ginseng.

In another embodiment of the food supplement of the invention there is provided a food supplement being in powder or granular form.

In another broad aspect, the present invention provides the use of said food supplement for supplementing bodybuilding by using the food supplement according to the present invention to achieve an increase in muscle size and strength.

Accordingly, in an embodiment of the invention there is achieved an increase an athlete's lean muscle mass and strength, administering to the athlete the said food supplement.

In another aspect, the use according to the invention preferably require that the supplement is administered to the athlete on a daily basis, and preferably the food supplement is mixed with water to provide a liquid drink.

In another embodiment there is provided an increasing muscle mass and/or strength of an individual, by administering to the individual the said food supplement.

Other features and advantages of the present invention will become apparent from the following detailed description. It should be understood, however, that the detailed description and the specific examples while indicating preferred embodiments of the invention are given by way of illustration only. Various changes and modifications within the scope of the claims will become apparent to those skilled in the art from this detailed description.

### DETAILED DESCRIPTION OF THE INVENTION

The food supplements and their use may provide further and significant muscle size and strength enhancement or improvement in individuals as compared with supplements employing only proteins and/or amino acids. While it is expected that the supplements and their use of the present invention will be of importance to bodybuilders and other athletes, the supplements and their uses are not limited to those groups. Rather, any individual may use the supplements of the invention. Indeed, the supplements may have applications to all animals.

Although the present invention is not to be limited by any theoretical explanation, it is believed that nitric oxide plays an essential role in tonic and exercise-associated (e. g., recovery from exercise) regulation of vasodilation and blood flow. Increased NO supports improved perfusion of skeletal muscle, which it is believed thereby stimulates oxidative metabolism, ATP and creatine phosphate biosynthesis, nutrient (e. g., amino acid) delivery and utilization for muscle (e. g., myofibrillar) protein synthesis, sarcomere addition, glucose delivery and uptake, glycogen synthesis, creatine delivery and uptake, creatine phosphate synthesis, fat loss regulation of nutrient-mediated insulin secretion, and nitrogen retention. The supplements according to the present invention are preferably based on a unique whey-protein system fortified with additional amino acids to further enhance the rapid absorption (i. e., of a-amino nitrogen) characteristics of whey which lend to its potent protein anabolic effects. Thus, combined with the metabolic effects of increased NO function, the supplements of the present invention effectively enhance the muscle protein anabolic effects of a source of amino acids, which is any source of protein, more preferably whey.

The food supplements and their uses provide further and significant size and strength enhancement through the role of certain substances which mimic and/or increase the sensitivity of insulin. Although, as already stated, the present invention is not to be limited by any theoretical explanation, it is believed that the family of substances known as the inositols, are physiological precursors to membrane protein anchors known as glycosylphatidylinositols (GPI). As used herein, "inositols"includes myo-inositol, cis-inositol, epi-inositol, allo-inositol, muco-inositol, neo-inositol, scyllo-inositol, d-chiro-inositol,1-chiro-inositol, d-pinitol and myo-inositol. Hydrolysis of GPI releases in inositolphosphoglycans which are water soluble mediators of insulin signalling events. Accordingly, increased availability of these GPI precursors, increases insulin sensitivity and these GPIs can trigger insulin signalling pathways and events independent of insulin thereby mimicking the effects of insulin. The pathways and events which are mimicked include, but are not limited to amino acid transport, protein synthesis, gene transcription, mRNA translation initiation, glucose uptake, glycogen storage, steroid hormone synthesis, and can thereby increase the development of muscle cells. Consequently, the said supplements of the invention may provide further and significant muscle size and strength enhancement as compared with supplements employing only proteins or amino acids.

The food supplements described herein comprise the compounds specifically identified and any derivatives thereof, for example a salt or ester. Suitable salts include, but are not limited to, alkali and alkaline earth metal salts, for example sodium, potassium or calcium salts, while suitable esters include, but are not limited to, alkyl esters, for example, methyl, ethyl or propyl esters, or lactone esters.

As used herein "source of amino acid" means protein. Throughout the present specification, as a part of the food supplement of the invention whey protein or a derivative thereof are identified as the preferred source of amino acids or protein. Commercially available whey protein derivatives include WPI 97, Whey Peptides, WPC 80, and ION EXCHANGE whey protein. However, as will be readily appreciated by those skilled in the art, other sources of protein include milk protein, casein, any of the albumins including chicken egg albumin, and soy, may be used as a source of amino acids.

As will be readily appreciated by those skilled in the art, the said supplement is not limited to only one substance which by itself may increase nitric oxide, insulin output, insulin secretion, insulin sensitivity, and one source of amino acids. Indeed the present invention provides for combinations of substances and sources of amino acids, in differing amounts.

The food supplement compositions of the present invention may be provided in a variety of formats, for example, in liquid form, powder form, or protein bar form. Powders are preferable and are prepared to be suitable for mixing with water or other liquids. The food supplement compositions in powder or granular form may be provided in accordance with customary processing techniques, for example as spray dried powders, or the like.

According to one embodiment, the food supplement compositions of the present invention are delivered in powder on a per one "scoop" basis. As used herein, one "scoop" is approximately 28 g of supplement. According to one embodiment, one "scoop" comprises: ginseng from 1mg-3000mg; myo-inositol, from 1mg-2000mg; d-chiro-inositol, from 1mg-2000mg; and glucomannan, from 10mg-4000mg; and a source of amino acids, which is a protein, most preferably whey.

According to another embodiment a food supplement composition comprises, when delivered in powder form per one "scoop": ginseng about 150mg to about 1500mg; myo-inositol about 100mg to about 2000mg; and glucomannan from about 25mg to about 2000mg; and a source of amino acids which is a protein, most preferably whey.

According to another embodiment the food supplement composition comprises, when delivered in powder form per one "scoop": ginseng from about 50mg to about 500mg; glucomannan from about 50mg to about 1000mg; and myo-inositol, from about 200mg to about 1000mg, and a source of amino acids which is a protein, most preferably whey.

The food supplement compositions according to the present invention may further contain additional components to further increase the speed and or ease with which the substances enter the bloodstream and subsequently possibly impact the muscle tissue, or to otherwise enhance the effects of nitric oxide in the body. For example, additional amino acids may be included in the food supplement compositions. Suitable amino acids include, but are not limited to, glutamine, alanine, taurine, carnitine, acetyl-L-carnitine, and the like. These additional amino acids may stimulate cell volumization and protein synthesis and therefore provide further advantages to increasing muscle strength and/or size. These amino acids may be employed individually or in various combinations and in amounts customary in the art, for example in the range of from about 0.01 mg to about 1000 mg per gram of food supplement depending upon the amount of protein, or peptides, derived from whey or other source of amino acid in the supplement.

The food supplement compositions can also contain ascorbic acid (vitamin C), for example in amounts equal to or exceeding the recommended minimum daily requirements. Another component for possible use in the food supplements of the present invention comprises beta-hydroxy, beta-methyl butyrate (HMB), in amounts known in the art.

The food supplement compositions may further comprise natural and/or artificial flavouring components, dyes or other colouring additives, preservatives and other conventional food supplement additives known in the art.

### Methods of Use of Supplements

The food supplements according to the present invention may be used for supplementing the diet of an athlete, and/or for enhancing an athlete's muscle size or strength. The food supplement compositions of the present invention are particularly advantageous for creating an increased anabolic environment and obtaining extra growth in lean muscle mass and strength. Accordingly, the present invention provides the use of an effective amount of a food supplement according to the present invention for supplementing bodybuilding in an athlete. Administration of an "effective amount" of the supplements and substances of the present invention is defined as an amount effective, at dosages and for periods of time necessary to achieve the desired result. The effective amount of the supplements of the invention may vary according to factors such as the age, sex, and weight of the athlete. Dosage regima may be adjusted to provide the optimum response: Several divided doses may be administered daily or the dose may be proportionally reduced as indicated by the exigencies of the individual athlete situation.

As just stated, the amount of the food supplement composition which is administered to the diet of the athlete may vary depending on the desired effect, the body weight and characteristics of the athlete, and the like. For example, in preferred methods for supplementing the diet of an athlete and/or for enhancing an athlete's muscle size or strength, from about 0.5 to about 10 scoops of a supplement according to present invention are administered to the diet of the athlete on a daily basis.

In more preferred embodiments of these methods, from about 1 to about 6 scoops of a supplement according to the present invention are administered to the diet of the athlete on a daily basis. The number of scoops and frequency per day will depend upon the composition of the scoop, as outlined in variations set out above.

As will be readily appreciated, a food supplement in accordance with the present invention may be administered in a single serving or in multiple servings spaced throughout the day. In a preferred embodiment, a food supplement in accordance with the present invention may be administered once in the morning, once immediately or shortly after training and once in the evening on a daily basis.

In order to maximize the effects of a food supplement according to the present invention, in enhancing muscle size and/or strength, it is preferred that the food supplement is administered to the diet of the athlete immediately following an exercise period. On non-workout days, the food supplement may be administered anytime during the day, although administering a first amount of a food supplement upon awakening or otherwise during the morning hours is preferred.

The food supplement compositions and methods of the invention are further illustrated in the following non-limiting examples. In the examples and throughout the present specification, parts and percentages are by weight unless otherwise specified.

### EXAMPLES

### EXAMPLE 1

A muscle size and/or strength enhancing regime is initiated and established so that the athlete consumes three servings of the following food supplement daily, with each serving being 1 scoop which is approximately 28 g of the supplement and comprising:

**Per 1 scoop:**

| | |
|---|---|
| Protein | 20g |
| Carbohydrates | 3g |
| Fiber | 1g |
| Sugar | 2g |
| Fat: | 1.5g |

These components may be comprised of the following:

| INGREDIENT | | | AMOUNT /SERVING |
|---|---|---|---|
| SynthePro^{™} A blend of the following constituting | | | 18g |
| | WPI 97 | | |
| | WHEY PEPTIDES | | |
| | WPC 80 | | |
| | ION EXCHANGE WHEY | | |
| | LACTOFERRIN | | |
| | METHIONINE | | |
| | LEUCINE | | |
| Nitroxen^{™} A blend of the following constituting | | | |
| | ARGININE | | |
| | GLYCOSIDAL SAPONINS | | |
| | FOLIC ACID | | 400mcg |
| Insulogen^{™} A blend of the following constituting | | | 200mg |
| | | MYO-INOSITOL, | |
| | | CIS-INOSITOL | |
| | | EPI-INOSITOL | |
| | | ALLO-INOSITOL | |
| | | MUCO-INOSITOL | |
| | | NEO-INOSITOL | |
| | | SCYLLO-INOSITOL | |
| | | D-CHIRO-INOSITOL | |
| | | L-CHIRO-INOSITOL | |
| | | INZITOL( (D-PINITOL) | |
| | ALPHA-LIPOIC ACID | | |
| | GLUCOMANNAN | | |
| | TAURINE | | 100mg |
| POTASSIUM PHOSPHATE | | | 100mg |
| VIT E | | | 30IU |
| NAC | | | 25mg |
| VIT B6 | | | 10.5mg |
| MAGNESIUM | | | 50mg |
| GUAR GUM | | | |

The rest of the volume comprising the following:
GLUTAMINE PEPTIDES
GLUTAMINE
ALPHA-KETO GLUTARATE
PHENYLALANINE
Creatine is included in the composition.

Each supplement serving is mixed with 6 ounces of cold water and the servings are administered approximately evenly spaced through the day, with a first serving in the morning, shortly after awakening, a second serving being consumed immediately after the athlete's exercise workout, and a third at a convenient time in the evening.

### EXAMPLE 2

A muscle size and/or strength enhancing regime as outlined in Example 1 is established so that the athlete consumes three servings of the food supplement daily, with each serving being 2 scoops which is approximately 56 g of the supplement.

### EXAMPLE 3

Where the regime in Example 1 or 2 is established, the athlete continues this regime on a day-to-day basis. As in Example 1 or 2, the serving is mixed with cold water to form a liquid drink. Alternatively, the food supplements may be combined with other liquid drinks or foods as desired. This regime is intended to last for the full term dumg which an athlete is working out. The servings and their timing should also be consumed on non-workout days (with the second serving being taken mid-day) in order to maintain enhanced muscle size and/or strength.

The servings set forth in these examples are designed for a 2000 calorie diet. Daily values may be increased or decreased depending on the calorie needs of individual athletes, and/or body weights of individual athletes.

### EXAMPLE 4 (not in accordance with the invention)

Thirty-six experienced weight trained males were randomly assigned (double-blind) to one of three experimental groups: 1. a dietary supplement of a whey protein blend, comprising whey protein isolate 97%, creatine, glycosidal saponins, arginine, and glucomannan (WI) - 1.2 g/kg/d whey protein, and 0.1 g/kg/d creatine; pure whey protein (W) -1.2 g/kg/d; or a placebo (P) - 1.2 g/kg/d maltodextrin. Pre and post test measures consisted of 1-RM bench press, 1-RM squat, Biodex isokinetic leg extension power (ILP), and body composition as assessed by dual energy x-ray absorptiometry (DEXA). Training involved a 4-day split routine of high volume, heavy load periodized workouts with sets and reps varying from 4-5 sets and 4-10 reps. Each group experienced a significant change from baseline measure (p<0.05). Post hoc analysis revealed that the WI group demonstrated greater increases in lean muscle mass (+4.0 kg), 1-RM bench press (+16 kg), and ILP (+23 J/s) than W and P (p<0.05). The W group had a significantly greater increase in lean mass (+2.3 kg) and ILP (+18 J/s) than the P group (p<0.05). These findings indicate that whey protein supplementation when combined with resistance training for 6-weeks results in significant gains in muscular power and lean muscle mass, and when the whey protein is combined with other potentially ergogenic ingredients, even larger gains in muscle mass, 1-RM strength, and power occur.

### EXAMPLE 5

36 trained males resistance-trained for 42 days and were divided into three equally sized groups and the groups' diets supplemented with one of:
Group 1 = the Supplement of Example 1 (1.2 g/kg/d), which included creatine
Group 2 = Whey Protein [WP] (1.2 g/kg/d)
Group 3 = Placebo [PL] (1.2 g/kg/d maltodextrin)

### Results

The following is illustrative of the results for individuals in Group 1: Subject 11a gained 14.5 *lbs* total body mass and 12.8 lbs of lean mass and added 60 lbs to his bench press and 90 lbs to his squat. Finally this individual lost 2.4 *lbs* of bodyfat & decreased bodyfat by 2%. With respect to results as between groups, Group 1 gained 147% more total body mass than Group 2 on WP, and gained 74% more lean tissue mass than Group 2. As well, Group 1 gained 270% more total body mass than placebo group Group 3. Further, Group 1 gained 344% more lean tissue mass than Group 3 and increased lean tissue mass (LTM) by 4.0 kg (8.8 *lbs*)*.* The bench press strength gains of Group 1 were significantly greater than those of Group 2 after 42 days. Finally, Group 1's bench press strength was increased by approximately 16 kg (35.2 lbs) after 42 days.

### Conclusion

The mass and strength gains of Group 1 were greater than those found in group 2 in weight-trained subjects.

### EXAMPLE 6

The purpose of this study was to evaluate the effects of two different types of whey protein supplements on body mass, body composition and exercise performance over 28 days, in resistance trained adults. In a double blind trial, 24 health athletic adults males were randomized to receive either a whey protein concentrate/isolate based product (Group 1: the Supplement of Example 1 which included creatine) or whey protein concentrate (WPC 80; Group 2). Both groups ingested 120 grams of supplemental protein daily. Each subject followed a diet that restricted food protein intake to 2.0 gm/kg/d. Total protein intake was -3.3 gm/kg/d with supplementation. In addition, all participants engaged in hypertrophy resistance exercise under the guidance of an exercise physiologist. At baseline, day 14 and 28, each subject underwent body composition and exercise testing. By day 28, Group 1 gained a statistically significant amount of body mass compared to Group 2 [1.28 vs. - 0.02 kg; p=0.043]. In terms of lean body mass (LBM) by day 14, Group 1 increased significantly compared to Group 2 (1.49 vs. -0.31 kg; p=0.01). By day 28 the intragroup change for Group 1 was significant [1.25 kg; p<0.01], while the change for Group 2 was insignificant. On day 14 Group 1 experienced a greater reduction in body fat compared to Group 2 (-1.06% vs. 0.53%; p=0.04). For exercise, there were no significant differences between the two groups however, both groups improved significantly over the 28 days (bench and leg press 1 rep maximum and repetitions until failure both at 1x and 2x body weight respectively). The results of this study indicate Group 1 gained more mass over 28 days than Group 2.

### EXAMPLE 7

For this study 24 trained males followed a control diet (2.0g protein/day) and resistance-trained for 28 days and supplemented with one of:
Group 1 = Supplement of Example 1 which included creatine (120g/day)
Group 2 = Whey Protein Concentration [WPC] (120g/day).

### Results

The lean body mass (LBM) gains of Group 1 were significantly greater than those of Group 2 on WPC as determined after 14 days (1.49 vs. -0.31 kg). It was also observed that Group 1 had significantly increased lean body mass (LBVM)(by 1.25 kg) after 28 days. Indeed, Group 1 had gained 2400% more LBM than WPC after 28 days. In addition to these gains, the total body mass gains (TBM) of Group 1 were significantly greater than Group after 28 days (1.28 vs. -0.02 kg). Finally, the % body fat loss was significantly greater in Group 1 than found for Group 2 after 14d (-1.06 vs. +0.53%). Three individual results exemplify the results of members from Group 1: After 14 days subject SV had an increase in LBM of 8.6 *lbs;* subject DK had an increase in 1 RM bench press of 25.3 lbs; and subject RB had an increase in 1 RM leg press of 90.2 *lbs.*

### Conclusion

Group 1 individuals experienced greater mass gains than those members of Group 2 (weight-trained subjects of both Groups).

The examples and embodiments set forth in the present application are provided only to illustrate various aspects of the invention and additional embodiments and advantages of the food supplements and methods of the present invention will be apparent to those skilled in the art.

## Claims

1. Use of a source of amino acids which is a protein, and of at least one substance which increases nitric oxide production in the body selected from the group consisting of ginseng, L-arginine, N-acetyl cysteine, glucomannan, and folic acid, and of a compound which is selected from the group consisting of myo-inositol, d-myo-inositol, cis-inositol, epi-inositol, allo-inositol, muco-inositol, neo-inositol, scyllo-inositol, d-chiro-inositol, 1-chiro-inositol and d-pinitol for preparation of a food supplement for at least one of improving or increasing lean muscle mass and/or strength.

2. The use according to claim 1, whereby the administration of the food supplement is daily.

3. The use according to claim 1 or 2, wherein said protein comprises a whey protein or a whey protein derivative.

4. The use according to any one of claims 1 to 3, wherein said food supplement is approximately 28 g in one embodiment.

5. The use according to any one of claims 1 to 4, wherein the food supplement comprises a carbohydrate.

6. The use according to any one of claims 1 to 5, wherein the food supplement comprises protein in an amount of 20 g.

7. The use according to any one of claims 1 to 6, wherein the food supplement comprises fat in an amount of 1.5 g.

8. The use according to any one of claims 1 to 7, whereby the administration of the food supplement is 0.5 to 10 scoops daily, preferred one to six scoops daily.

9. The use according to any one of claims 1 to 8, whereby the administration of the food supplement is immediately following an exercise period.

10. The use according to any one of claims 1 to 9, wherein said food supplement is in powder form.

11. The use according to claim 10, wherein said powder is mixed with water for administration as a liquid.

12. The use according to any one of claims 1 to 11, wherein said food supplement further comprises one or more compounds selected from the group consisting of glutamine, alanine, taurine, carnitine, and acetyl-L-carnitine.

13. A food supplement for at least one of improving or increasing lean muscle mass and/or strength, comprising a source of amino acids and at least one substance which increases nitric oxide production in the body selected from the group consisting of ginseng, L-arginine, N-acetyl cysteine, glucomannan, and folic acid, wherein the source of amino acids is a protein; and further comprising a compound which mimics or enhances insulin activity which is selected from the group consisting of myo-inositol, d-myo-inositol, cis-inositol, epi-inositol, allo-inositol, muco-inositol, neo-inositol, scyllo-inositol, d-chiro-inositol, 1-chiro-inositol and d-pinitol.

14. The food supplement according to claim 13 for daily administration.

15. The food supplement according to claim 13 or 14, wherein said protein comprises a whey protein or a whey protein derivative.

16. The food supplement according to any one of claims 13 to 15, wherein said food supplement is approximately 28 g in one embodiment.

17. The food supplement according to any one of claims 13 to 16, wherein the food supplement comprises a carbohydrate.

18. The food supplement according to any one of claims 13 to 17, wherein the food supplement comprises protein in an amount of 20 g.

19. The food supplement according to any one of claims 13 to 18, wherein the food supplement comprises fat in an amount of 1.5 g.

20. The food supplement according to any one of claims 13 to 19 for 0.5 to 10 scoops per daily administration.

21. The food supplement according to any one of claims 13 to 20, wherein said food supplement is a powder.

22. The food supplement according to claim 21, wherein said powder is mixed with water for administration as a liquid.

23. The food supplement according to any one of claims 13 to 22, wherein said food supplement further comprises one or more compounds selected from the group consisting of glutamine, alanine, taurine, carnitine, and acetyl-L-carnitine.

24. Use of a food supplement of any one of claims 13 to 23 for at least one of improving or increasing lean muscle mass and/or strength, said use not being a therapeutic use.

## Patentansprüche

1. Verwendung einer Quelle von Aminosäuren, die ein Protein ist, und mindestens einer Substanz, die die Produktion von Stickstoffmonoxid im Körper steigert, die aus der Gruppe ausgewählt wird, die aus Ginseng, L-Arginin, N-Acetylcystein, Glukomannan und Folsäure besteht, und einer Verbindung, die aus der Gruppe ausgewählt wird, die aus Myo-Inositol, D-myo-Inositol, Cis-Inositol, Epi-Inositol, Allo-Inositol, Muco-Inositol, Neo-Inositol, Scyllo-Inositol, D-chiro-Inositol, 1-chiro-Inositol und D-Pinitol besteht, zur Zubereitung eines Nahrungsmittelzusatzes zur Verbesserung und/oder Zunahme der fettarmen Muskelmasse und/oder Kraft.

2. Die Verwendung nach Anspruch 1, wodurch die Verabreichung des Nahrungsmittelzusatzes täglich erfolgt.

3. Die Verwendung nach Anspruch 1 oder 2, wobei besagtes Protein ein Molkeprotein oder ein Molkeproteinderivat umfasst.

4. Die Verwendung nach einem der Ansprüche 1 bis 3, wobei besagter Nahrungsmittelzusatz in einer Ausführungsform ca. 28 g beträgt.

5. Die Verwendung nach einem der Ansprüche 1 bis 4, wobei der Nahrungsmittelzusatz ein Kohlenhydrat umfasst.

6. Die Verwendung nach einem der Ansprüche 1 bis 5, wobei der Nahrungsmittelzusatz Protein in einer Menge von 20 g umfasst.

7. Die Verwendung nach einem der Ansprüche 1 bis 6, wobei der Nahrungsmittelzusatz Fett in einer Menge von 1,5 g umfasst.

8. Die Verwendung nach einem der Ansprüche 1 bis 7, wodurch täglich 0,5 bis 10 Löffel, vorzugsweise täglich ein bis sechs Löffel des Nahrungsmittelzusatzes verabreicht werden.

9. Die Verwendung nach einem der Ansprüche 1 bis 8, wodurch die Verabreichung des Nahrungsmittelzusatzes unmittelbar nach einer Trainingseinheit erfolgt.

10. Die Verwendung nach einem der Ansprüche 1 bis 9, wobei besagter Nahrungsmittelzusatz in Pulverform vorliegt.

11. Die Verwendung nach Anspruch 10, wobei besagtes Pulver mit Wasser gemischt wird, um als Flüssigkeit verabreicht zu werden.

12. Die Verwendung nach einem der Ansprüche 1 bis 11, wobei besagter Nahrungsmittelzusatz außerdem eine oder mehrere Verbindungen umfasst, die aus der Gruppe ausgewählt wird/werden, die aus Glutamin, Alanin, Taurin, Carnitin und Acetyl-L-carnitin besteht.

13. Ein Nahrungsmittelzusatz zur Verbesserung und/oder Zunahme der fettarmen Muskelmasse und/oder Kraft, umfassend eine Quelle von Aminosäuren und mindestens eine Substanz, die die Produktion von Stickstoffmonoxid im Körper steigert, die aus der Gruppe ausgewählt wird, die aus Ginseng, L-Arginin, N-Acetylcystein, Glukomannan und Folsäure besteht, wobei die Quelle von Aminosäuren ein Protein ist, und außerdem umfassend eine Verbindung, die die Insulinwirkung nachahmt oder steigert, die aus der Gruppe ausgewählt wird, die aus Myo-Inositol, D-myo-Inositol, Cis-Inositol, Epi-Inositol, Allo-Inositol, Muco-Inositol, Neo-Inositol, Scyllo-Inositol, D-chiro-Inositol, 1-chiro-Inositol und D-Pinitol besteht.

14. Der Nahrungsmittelzusatz nach Anspruch 13 zur täglichen Verabreichung.

15. Der Nahrungsmittelzusatz nach Anspruch 13 oder 14, wobei besagtes Protein ein Molkeprotein oder ein Molkeproteinderivat umfasst.

16. Der Nahrungsmittelzusatz nach einem der Ansprüche 13 bis 15, wobei besagter Nahrungsmittelzusatz in einer Ausführungsform ca. 28 g beträgt.

17. Der Nahrungsmittelzusatz nach einem der Ansprüche 13 bis 16, wobei der Nahrungsmittelzusatz ein Kohlenhydrat umfasst.

18. Der Nahrungsmittelzusatz nach einem der Ansprüche 13 bis 17, wobei der Nahrungsmittelzusatz Protein in einer Menge von 20 g umfasst.

19. Der Nahrungsmittelzusatz nach einem der Ansprüche 13 bis 18, wobei der Nahrungsmittelzusatz Fett in einer Menge von 1,5 g umfasst.

20. Der Nahrungsmittelzusatz nach einem der Ansprüche 13 bis 19 zur täglichen Verabreichung von 0,5 bis 10 Löffeln.

21. Der Nahrungsmittelzusatz nach einem der Ansprüche 13 bis 20, wobei besagter Nahrungsmittelzusatz ein Pulver ist.

22. Der Nahrungsmittelzusatz nach Anspruch 21, wobei besagtes Pulver mit Wasser gemischt wird, um als Flüssigkeit verabreicht zu werden.

23. Der Nahrungsmittelzusatz nach einem der Ansprüche 13 bis 22, wobei besagter Nahrungsmittelzusatz außerdem eine oder mehrere Verbindungen umfasst, die aus der Gruppe ausgewählt wird/werden, die aus Glutamin, Alanin, Taurin, Carnitin und Acetyl-L-carnitin besteht.

24. Verwendung eines Nahrungsmittelzusatzes nach einem der Ansprüche 13 bis 23 zur Verbesserung und/oder Zunahme der fettarmen Muskelmasse und/oder Kraft, wobei besagte Verwendung keine therapeutische Verwendung ist.

## Revendications

1. Utilisation d'une source d'acides aminés qui est une protéine, et d'au moins une substance qui augmente la protection d'oxyde nitrique dans le corps, sélectionnée dans le groupe constitué de ginseng, L-arginine, N-acétyl cystéine, glucomannan et acide folique, et d'un composé qui est sélectionné dans le groupe constitué de myo-inositol, d-myo-inositol, cis-inositol, épi-inositol, allo-inositol, muco-inositol, néo-inositol, scyllo-inositol, d-chiro-inositol, 1-chiro-inositol et d-pinitol pour la préparation d'un complément alimentaire pour au moins un but parmi l'amélioration ou l'augmentation de la masse maigre et/ou de sa résistance.

2. Utilisation selon la revendication 1, par laquelle l'administration du complément alimentaire est quotidienne.

3. Utilisation selon la revendication 1 ou 2, où ladite protéine comprend une protéine lactosérique ou un dérivé de protéine lactosérique.

4. Utilisation selon l'une quelconque des revendications 1 à 3, où ledit complément alimentaire est d'environ 28 g dans un mode de réalisation.

5. Utilisation selon l'une quelconque des revendications 1 à 4, où le complément alimentaire comprend un glucide.

6. Utilisation selon l'une quelconque des revendications 1 à 5, où le complément alimentaire comprend une protéine à raison d'une quantité de 20 g.

7. Utilisation selon l'une quelconque des revendications 1 à 6, où le complément alimentaire comprend de la graisse à raison d'une quantité de 1,5 g.

8. Utilisation selon l'une quelconque des revendications 1 à 7, par laquelle l'administration du complément alimentaire est de 0,5 à 10 mesurettes quotidiennes, de préférence une à six mesurettes quotidiennes.

9. Utilisation selon l'une quelconque des revendications 1 à 8, par laquelle l'administration du complément alimentaire suit immédiatement une période d'exercice.

10. Utilisation selon l'une quelconque des revendications 1 à 3, où ledit complément alimentaire est sous forme de poudre.

11. Utilisation selon la revendication 10, où ladite poudre est mélangée à de l'eau pour une administration sous forme de liquide.

12. Utilisation selon l'une quelconque des revendications 1 à 11, où ledit complément alimentaire comprend également un ou plusieurs composés sélectionnés dans le groupe constitué de glutamine, alanine, taurine, carnitine et actéyl-L-carnitine.

13. Complément alimentaire pour au moins un but parmi l'amélioration ou l'augmentation de la masse maigre et/ou de sa résistance, comprenant une source d'acides aminés et au moins une substance qui augmente la production d'oxyde nitrique dans le corps, sélectionnée dans le groupe constitué de ginseng, L-arginine, N-acétyl cystéine, glucomannan et acide folique, où la source d'acides aminés est une protéine ; et comprenant un composé qui simule ou augmente l'activité de l'insuline et qui est sélectionné dans le groupe constitué de myo-inositol, d-myo-inositol, cis-inositol, épi-inositol, allo-inositol, muco-inositol, néo-inositol, scyllo-inositol, d-chiro-inositol, 1-chiro-inositol et d-pinitol.

14. Complément alimentaire selon la revendication 13 pour une administration quotidienne.

15. Complément alimentaire selon la revendication 13 ou 14, où ladite protéine comprend une protéine lactosérique ou un dérivé de protéine lactosérique.

16. Complément alimentaire selon l'une quelconque des revendications 13 à 15, où ledit complément alimentaire est d'environ 28 g dans un mode de réalisation.

17. Complément alimentaire selon l'une quelconque des revendications 13 à 16, où le complément alimentaire comprend un glucide.

18. Complément alimentaire selon l'une quelconque des revendications 13 à 17, où le complément alimentaire comprend une protéine à raison d'une quantité de 20 g.

19. Complément alimentaire selon l'une quelconque des revendications 13 à 18, où le complément alimentaire comprend de la graisse à raison d'une quantité de 1,5 g.

20. Complément alimentaire selon l'une quelconque des revendications 13 à 19 pour 0,5 à 10 mesurettes par administration quotidienne.

21. Complément alimentaire selon l'une quelconque des revendications 13 à 20, où ledit complément alimentaire est une poudre.

22. Complément alimentaire selon la revendication 21, où ladite poudre est mélangée avec de l'eau pour une administration sous forme de liquide.

23. Complément alimentaire selon l'une quelconque des revendications 13 à 22, où ledit complément alimentaire comprend un ou plusieurs composés sélectionnés dans le groupe constitué de glutamine, alanine, taurine, carnitine et actéyl-L-carnitine.

24. Utilisation d'un complément alimentaire selon l'une quelconque des revendications 13 à 23 pour au moins un but parmi l'amélioration ou l'augmentation de la masse maigre et/ou de sa résistance, ladite utilisation n'étant pas une utilisation thérapeutique.
